(19) Europäisches Patentamt
European Patent Office
Office européen des brevets

(11) Publication number: 0 075 937
B1

(12) # EUROPEAN PATENT SPECIFICATION

(45) Date of publication of patent specification: **02.05.85**

(51) Int. Cl.⁴: **C 07 C 29/15,** B 01 J 31/20, C 07 C 31/20, C 07 C 31/04

(21) Application number: **82108998.4**

(22) Date of filing: **29.09.82**

(54) **Process for producing alcohols.**

(30) Priority: **30.09.81 US 307216**

(43) Date of publication of application: **06.04.83 Bulletin 83/14**

(45) Publication of the grant of the patent: **02.05.85 Bulletin 85/18**

(84) Designated Contracting States: **BE DE FR GB IT NL SE**

(56) References cited:
**EP-A-0 013 008**
**EP-A-0 033 425**
**US-A-4 315 994**

(73) Proprietor: **UNION CARBIDE CORPORATION**
**Old Ridgebury Road**
**Danbury Connecticut 06817 (US)**

(72) Inventor: **Dombek, Bernard Duane**
**1631 Woodvale Drive**
**Charleston 25314 West Virginia (US)**
Inventor: **Hart, Paul William**
**Route 2 Box 7**
**Alum Creek 25003 West Virginia (US)**
Inventor: **O'Connor, George Lawrence**
**844 Matthews Avenue**
**Charleston 25314 West Virginia (US)**

(74) Representative: **Wuesthoff, Franz, Dr.-Ing. et al**
**Patentanwälte Wuesthoff -v. Pechmann-**
**Behrens-Goetz Schweigerstrasse 2**
**D-8000 München 90 (DE)**

**EP 0 075 937 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to an improved process, and the catalyst which achieves this process, for making ethylene glycol and mono and polyhydric compound, e.g. methanol directly from synthesis gas, i.e., mixtures of hydrogen and carbon monoxide. More particularly, this invention achieves the production of ethylene glycol directly from synthesis gas using a ruthenium carbonyl complex catalyst and a rhodium carbonyl cocatalyst under process conditions which heretofore were regarded as being incapable of producing ethylene glycol with only a ruthenium containing catalyst or rhodium containing catalyst at selectivities and rates observed herein. This invention encompasses a process of producing such compounds directly from the reaction of synthesis gas in the presence of a stable ruthenium catalyst and a stable rhodium cocatalyst. The process of this invention is distinctive in the stability of the process, avoiding any significant loss of ruthenium and rhodium values from reaction. In addition, this process features a unique ruthenium catalyst and rhodium cocatalyst having rhodium in a mononuclear form.

Discussion of the prior art

The limited availability of petroleum sources and the cost of producing chemicals from petroleum sources has been steadily increasing. Many have predicted that significant oil shortages will arise in the future. Obviously, an alternative low cost source is needed to replace petroleum as the raw material for conversion into the valuable chemicals now derived from petroleum sources. Synthesis gas is one such source. It is one which can be effectively utilized in certain circumstances to make some of the valuable chemicals now derived primarily from petroleum sources.

The economic attractiveness of synthesis gas derives from the fact that it can be produced from non-petroleum sources. Synthesis gas is derived by the combustion of any carbonaceous material including coal, or any organic material, such as hydrocarbons, carbohydrates and the like. Synthesis gas has for a long time been considered a desirable starting material for the manufacture of a variety of chemicals. A number of chemicals have been made commercially from synthesis gas. Hydrocarbons have been made by the Fisher-Tropsch catalytic reaction. Methanol is commercially manufactured by a heterogeneous catalytic reaction from synthesis gas. Aldehydes and alcohols are made from the reaction of olefins and synthesis gas. If the production of chemicals from synthesis gas could be expanded in a commercial manner, then the present dependence upon petroleum sources as the basic raw material for chemicals would be greatly lessened, even though petroleum is itself an excellent raw material for making synthesis gas. Accordingly, intense interest in such processes has developed.

The search for a process using synthesis gas as the raw material has received much interest in the last five to ten years as is evident from a review of the patent and non-patent literature. A brief review of a portion of this literature follows:

U.S. Patent No. 3,833,634, describes a process for preparing glycols by reacting an oxide of carbon with hydrogen using a rhodium carbonyl complex catalyst. The examples of the patent compare the reaction of hydrogen and carbon monoxide in the presence of the desired rhodium containing catalyst and other metals. In Example 9 of the patent, the reaction was attempted with triruthenium dodecacarbonyl as the catalyst using tetrahydrofuran as the solvent with a reaction temperature of 230°C, for 2 hours, and "the product contained no polyhydric alcohol." Pruett and Walker failed to produce polyhydric alcohols when employing the ruthenium catalyst apparently because the conditions of reaction were not maintained long enough and/or with enough ruthenium containing catalyst to achieve the reaction to produce a detectable amount of a polyhydric alcohol such as ethylene glycol. Unquestionably, ruthenium is not as active a catalyst source to produce glycol as is rhodium under the conditions investigated.

U.S. Patent No. 2,535,060, describes a process for preparing monohydric alcohols by introducing carbon monoxide, hydrogen and a hydroxylated solvent into a reaction vessel and heating the mixture in the presence of a ruthenium-containing substance and an alkaline reagent which controls the pH within the range of 7 to 11.5, at a temperature within the range of 150° to 300°C under a pressure within the range of 200 to 1,000 bar.

Solid ruthenium dioxide is used in Examples 1 and 2 of the aforementioned Gresham patent. At column 2, lines 30—33 of the patent, the patentee states his belief that ruthenium dioxide is reduced *in situ* during the reaction. Example 1 compares the use of a number of solutes such as phosphoric acid, acidic phosphate buffer, no solutes at all, ammonia and sodium bicarbonate. In this example the solvent was water. In Example 2 of US—P 2535060, a number of alcohols were characterized as solvents.

In US—P 2535060 is stated that ruthenium and its compounds are "specific" in their effect upon this reaction and other catalysts "do *not* lead to straight chain primary alcohols under the conditions of this process". There is no indication that this process produced ethylene glycol.

US—P 2535060 should be contrasted with his earlier work described in U.S. Patent No. 2,636,046. In this patent, is described the production of polyfunctional oxygen-containing organic products including such compounds as ethylene glycol, glycerine, and the like.*

* Note the evaluation of this work by Rathke and Feder, J. Am. Chem. Soc., *100*, pp. 3623—3625 (May 24, 1978).

This is accomplished by the reaction of hydrogen with carbon monoxide in the presence of a solvent to produce glycol. According to this patent, the reaction of carbon monoxide with hydrogen must be at pressures of above 1,000 atmospheres and "particularly above a minimum of about 1,400 atmospheres in order to obtain the "polyfunctional oxygen-containing organic compounds... in excellent yield" (column 2, lines 9—17). The patent specifically states at column 2, lines 37—43, that:

"[I]n the hydrogenation of oxides of carbon at pressures of 1,000 atmospheres and below, virtually no polyfunctional compounds are produced. At pressures above 1,000 atmospheres and especially at pressures of about 1,500 to 5,000 atmospheres, preferably 2,000 to 5,000 atmospheres, polyfunctional compounds are obtained."

Though the examples of the patent describe only the use of cobalt catalyst, the patentee, at column 3, line 61, indicates that the catalyst may contain "cobalt, ruthenium, etc." According to the patentee, the most outstanding results are obtained by using a catalyst containing cobalt, especially compounds of cobalt which are soluble in at least one of the ingredients of the reaction mixtures.

The testing of other metals, i.e., other than rhodium, is reported by Roy L. Pruett, *Annals, New York Academy of Sciences*, Vol. 295, pages 239—248 (1977), at page 245, to determine the production of ethylene glycol from mixtures of carbon monoxide and hydrogen. These metals include cobalt, ruthenium, copper, manganese, iridium and platinum. Of these metals, only cobalt was found to have a slight activity, citing British Patent 665,698 which corresponds generally to the last mentioned U.S. Patent 2535060 stated that such slight activity with cobalt was "qualitatively" in agreement with the results obtained by Ziesecke, 1952, Brennstoff-Chem, *33*:385.

Prior to the filing of U.S. Patent No. 2,535,060 and subsequent to the filing of U.S. Patent No. 2,636,046, there was filed U.S. Patent No. 2,549,470, directed to a catalytic process for making monohydric straight chain alcohols and does not mention the production of ethylene glycol. The patent emphasizes the production of straight chain primary hydroxyalkanes having from 3 to 50 or more carbon atoms in the molecule. This, the patent states, is accomplished by introducing hydrogen, carbon monoxide, and a hydroxylated solvent into a reaction vessel, and heating the mixture in the presence of a catalyst of the class consisting of ruthenium metal, ruthenium oxide and ruthenium carbonyl, at a pressure within the range of 200 to 1,000 atmospheres and at a temperature within the range of 100° to 250°C. The liquid hydroxyl-containing reaction medium may be water or alcohol, preferably a primary hydroxyalkane having from 1—10 carbon atoms per molecule. According to the patentee, a substantial proportion of the reaction product usually consists of alcohols containing more than 6 carbon atoms per molecule. The patent goes on to state (column 1, line 50, et seq.):

"The reaction products usually contain virtually no hydrocarbons, acids, esters, or branched-chain alcohols. These results were entirely unexpected, in view of the existing knowledge of the catalytic reaction between carbon monoxide and hydrogen in the presence of alcohols and Group VIII metal catalysts."

U.S. Patent No. 3,579,566, is concerned with a process of reducing organic acid anhydrides with hydrogen in the presence of a Group VIII noble metal catalyst and a biphyllic ligand of phosphorus, arsenic or antimony. The process of Fenton bears a remarkable similarity to oxo processing conditions to produce aldehydes and alcohols (compare U.S. Patent No. 3,539,634, except that US—P 3579566 fails to supply an olefinic compound to the reaction. In the reaction an acid anhydride, such as acetic acid anhydride, is reduced to ethylidene diacetate in the presence of hydrogen and a rhodium halide or a mixture of palladium chloride and ruthenium trichloride catalyst, provided in combination with triphenylphosphine. Ethylene glycol diacetate is also observed. Carbon monoxide, which is added to some of the examples is described at column 2, lines 48—51, as follows: "If desired, a suitable *inert* gas, such as carbon monoxide can also be charged to the reaction zone...". (Emphasis added). Of particular significance is the fact that none of Fenton's examples produce a methyl ester, as are produced by the process of copending U.S. Patent Application S.N. 971,667, discussed below. Another point is that ethylidene diacetate can be thermally cracked to produce vinyl acetate, see column 1, lines 42—44 of US—P 3539566. It would seem possible that such occurred in Example 1 and it is further possible that acetic acid added to the vinyl acetate to form ethylene glycol diacetate.

An interesting exception to the previously reported inactivity of ruthenium catalysts to produce glycol is the high pressure (viz 1650—1750 bar) experiment reported by Fonseca, Jenner, Kiennemann, and Deluzarche, et al., High Pressure Science and Technology, 6th AIRAPT Conference (Chapt. "High Pressure Synthesis of Polyalcohols by Catalytic Hydrogenation of Carbon Monoxide"), pages 733—738 (1979), published by Plenum Press, New York (see also a discussion of the same work in Erdol und Kohle, *32*, 313 (1979)). The authors report the reaction in tetraglyme of a $CO:H_2$ (1:2 ratio) mixture at 1650—1765 bars, i.e., about 25,000 psi (1,757.6 $kg/cm^2$) and 230°C using triruthenium dodecacarbonyl and 2-pyridinol as a ligand, both in unstated amounts, for a period of 5 hours. The authors report a percent conversion of 12.9 (unstated basis), and percent yield of polyols of 3 (unstated basis), and percent selectivities as follows: ethylene glycol, 22.9; glycerine, 0; methanol, 16.1. However, in a manuscript entitled "Reactions $CO—H_2$ in Liquid Phase in Presence of Ruthenium Catalysts," by Jenner, Kiennemann, Bagherzadah, and Deluzarche, (React. Kim. Catal. Letters, *15*, 103 (1980)). It is stated that with respect to the above experiment and relating to the catalytic activity of ruthenium that "We never could reproduce the run with $Ru_3(CO)_{12}$ when operating in a vessel which has not been in contact with any rhodium catalyst. We suspect that in the former run, the

formation of ethylene glycol was due to catalysis with metallic sediments of rhodium encrusted on the wall of the vessel (we showed that ethylene glycol is produced in appreciable yield with rhodium foam)".* It is clear that the authors disclosed only a rhodium catalyst, i.e. verify the results of U.S. Patent No. 3,833,634. In United States Patent 4,170,605 reports in Examples I and II the reaction in 1-propanol of synthesis gas ($CO:H_2=1:1$) at 25,000 psig and at 230°C using ruthenium tris(acetylacetonate) and 2-hydroxypyridine, the latter being the same ligand employed by Fonseca, et al *supra*, for a period of 2 and 3 hours, respectively. In Example 1 of US—P 41 70605 is reported the production of 4 grams of product** containing (mole percent basis): ethylene glycol, 57; and methanol 25. In Example II, 7 grams of product** are reported containing 66 and 16 mole percent of ethylene glycol and methanol, respectively.

W. Keim et al., (Journal of Catalysis, *61*, 359 (1980)) has reported that reactions of $Ru_3(CO)_{12}$ under very high pressures (2,000 bar) produce mainly methanol and methyl formate, but traces of glycol (0.8 to 1.2 percent of the total products) were also seen. In one experiment a small amount of ethanol was detected. No glycerine was observed in these reactions.

In a recent report (J. Am. Chem. Soc., *101*, 7419 (1979).), J. S. Bradley of Exxon Corporation reported the production of methanol and methyl formate at a selectivity greater than 99% without hydrocarbon products detected, by the reaction of synthesis gas ($H_2:CO=3:2$) under pressures on the order of 1,300 atmospheres and at temperatures around 270°C using a Ru catalyst. Bradley observed that no ethanol, ethylene glycol, or acetates formed. Compare this result with that found in US—P 3833634, *supra*, and the work of Fonseca et al. and US—P 4170605, *infra*.

As pointed out above, ethylene glycol can be produced directly from a mixture of hydrogen and carbon monoxide using a rhodium carbonyl complex as a catalyst. The literature describes (see U.S. Patent No. 3,957,857, that a desirable rhodium compound can be in the form of a rhodium carbonyl cluster compound, particularly one which exhibits a particular 3-band infrared spectral pattern. There has been a substantial amount of work done on the formation of ethylene glycol from mixtures of hydrogen and carbon monoxide in the presence of rhodium carbonyl clusters, such as is described in United States Patent Nos. 3,833,634; 3,878,214; 3,878,290; 3,878,292; etc. to name but a few.

In addition, the use of divalent metal salts of dodecametal triaconta carbonyls to make ethylene glycol is suggested in U.S. Patent Nos. 3,989,799 wherein $Ru(Rh_yIr_{12-y}(CO)_{30})$ is disclosed. As is obvious from the patent, the ruthenium triaconta carbonyl salt never contains a stoichiometric excess of ruthenium and is not a mixed metal catalyst as employed in the instant process.

The above discussion provides a brief characterization of the technology heretofore published or filed upon which relates to the direct production of ethylene glycol from mixtures of carbon monoxide and hydrogen or the production of monohydric alcohols from the direct reaction of hydrogen and carbon monoxide in the presence of a ruthenium or a rhodium catalyst. For the purposes of the discussion and descriptions contained herein, mixtures of hydrogen and carbon monoxide, regardless of the amount of each present, will be characterized, for the sake of convenience, as "synthesis gas". Thus, mole ratios of hydrogen to carbon monoxide of e.g. 40 to 1 and 1 to 40 are arbitrarily classified as "synthesis gas". Where the molar ratio of one or the other is significant to the invention herein described, then specific reference to the desired molar ratio will be made.

From the EP—A 00 33 425 is known the production of ethylene glycol, methanol, ethanol and/or esters thereof from synthesis gas which comprises contacting a mixture of CO and $H_2$ with a catalyst at elevated temperature in liquid medium. The catalyst comprising ruthenium and rhodium, preferably as carbonyl complex, and optionally a promoter selected from the group of oxides, hydroxides or salts of a metal of the group Ia, IIa oder IIb.

Brief description of the figures

Figure 1 graphically depicts examples 1 to 5, discussed hereinafter.

Figure 2 graphically depicts examples 6 to 10, discussed hereinafter.

This invention relates to the process and for making alkanols, including methanol and ethylene glycol, directly from the reaction of hydrogen and carbon monoxide by the reaction of hydrogen and carbon monoxide in the liquid phase in the presence of a ruthenium carbonyl catalyst, a rhodium carbonyl cocatalyst and an alkali metaliodid as promoter, e.g., at a temperature and pressure sufficient to form said alkanols. The inventive process provides a rate of formation for ethylene glycol heretofore unobserved for such systems with a decrease in the rate of formation of methanol as well as minor amounts of ethanol, methyl formate and acetaldehyde. The process of this invention provides the capability of a low cost route to alkane polyols, especially ethylene glycol.

The process of this invention is carried out with the ruthenium carbonyl complex and the rhodium carbonyl catalyst dissolved in a solvent, even though such compounds may exist during the reaction in

* This report may be relevant to the reports by Williamson et al. (infra) and Keim et al. (infra).

** Included in the 4 and 7 grams of product are trace amounts of water and methylformate as well as 16 mole percent (Example I) and 15 mole percent (Example II) of propylformate. The latter compound would appear to be derived from 1-propanol initially present in the reaction mixture, rather than a synthesis gas-derived product.

more than one liquid phase. In this sense, the reaction is termed a homogeneous liquid phase reaction. There may be more than one such phase existing in the reaction zone but the ruthenium carbonyl compound and the rhodium carbonyl compound that exist are always dissolved in at least one of such phases and are always in a dissolved liquid state. The problem with heterogeneous ruthenium catalysis in the reaction zone is that such will induce the Fischer-Tropsch reaction result in the formation of hydrocarbons and/or a variety of oxygenated hydrocarbons having a variety of molecular weights with low selectivity to any one compound. In fact the presence of such products suggests that undissolved ruthenium is present. Such problems are not observed in the instant process. Starting from the process according to the afore-mentioned EP—A 00 33 425 the reaction of synthesis gas takes place in a homogeneous liquid phase in the presence of a ruthenium carbonyl catalyst, a rhodium carbonyl cocatalyst, a promoter and a solvent at 50 to 400°C and a pressure of 34.5 to 862 bar and is characterized in that a combination of a non-ionic polar solvent and an alkali metal halide promoter is used.

The role of ruthenium in the reaction is believed to be the initiation of the reaction between carbon monoxide and hydrogen to form an intermediate species such as a formyl group (—CHO). Rhodium in the reaction is believed to function in converting such formyl groups to ethylene glycol.

The process of this invention is distinctive in the selection of materials which comprise the homogeneous liquid phase mixture, the reaction parameters and the stability of the ruthenium-containing catalyst and rhodium-containing cocatalyst in most cases, indeed, in all cases studied. As with any new technology, this process has undergone evolutionary changes and its further examination will undoubtedly bring about more changes, most likely in the form of additional or substitutional steps and/or materials.

The process of the invention is carried out in the presence of an iodide promoter. A promoter, in the context of this invention, is a material provided to the reaction which provides a promotional effect in that it enhances the production (viz., rate, yield or efficiency) of any of the products, or it improves the selectivity of the reaction toward ethylene glycol rather than methanol, or it improves the selectivity of the reaction to ethylene glycol rather than ethanol irrespective of the amount of methanol produced, or it helps to reduce any small loss of the ruthenium-containing and rhodium-containing catalyst which might occur during the reaction.

The selections of solvent and the promoter are not narrowly limited yet there appears to be some degree of cooperation that each imparts to the success of the process and the selection of one often dictates the selection of the other in order to maximize the benefits of the invention.

It is found necessary that there be used a solvent that is capable of maintaining the chosen ruthenium catalyst, rhodium cocatalyst and the promoter in the homogeneous liquid phase mixture throughout the reaction. This appears to be the prime function of the solvent.

The catalyst of this invention has as one component thereof a ruthenium compound (or compounds) which contains carbon monoxide directly bonded to ruthenium (ruthenium carbonyl). The second component is a rhodium carbonyl cocatalyst.

The ruthenium compound which is provided to the reaction is not necessarily in a form which will effectively catalyze the reaction even if it contains a carbon monoxide ligand bonded to it. Ruthenium compounds such as ruthenium salts, oxides and carbonyl clusters may be introduced to the reaction in a condition which allows them to be solubilized, and under the conditions of the reaction they are converted into a catalyst which effectively catalyzes the reaction when present with the rhodium catalyst. That is why the catalyst is defined in terms of products made by the process. The composition and structure of the ruthenium carbonyl complex (or complexes) which catalyzes the desired reaction when present with the rhodium cocatalyst is not specifically known. It may be a monoruthenium or polyruthenium compound. Illustrative of polyruthenium compound are the well-known cluster compounds of ruthenium. However, the addition of a cluster containing only a carbonyl ligand such as $Ru_3(CO)_{12}$ does not alone create the ruthenium carbonyl component of the catalyst and as such cause the catalytic reaction when not present with the rhodium carbonyl component. Some modification of its structure is needed. Factors important in achieving the catalyst are the reaction parameters, the choice of solvent and, optionally, the Lewis base promoter that one employs. Because varied reaction conditions and solvents, with and without promoters, result in different amounts of the desired products of the process, and different rates, efficiencies and/or yields, it is presumed that each provides a different and distinct catalytic environment.

The ruthenium-containing substances which may be employed in the practice of this invention to form the ruthenium catalyst under process conditions encompass those which are described, for example, in U.S. Patent No. 2,535,060 at column 2, starting at line 38 to line 48, and ruthenium carbonyl compounds. It is not advisable to place ruthenium compounds or substances on a support material for use in the process of this invention because such offers no benefits over solubilizing such ruthenium compounds in combination with the rhodium cocatalyst, solvent and, optionally, the Lewis base promoter. Moreover, ruthenium deposited on a support material can be expected to be solubilized in the homogeneous liquid phase reaction system of this invention as it is contacted with carbon monoxide. Even ruthenium metal in the presence of the solvent, carbon monoxide and hydrogen can be converted to a ruthenium carbonyl complex which is soluble. Ruthenium oxides, such as dioxide, sesquioxide, or tetraoxide, are capable under appropriate conditions of being solubilized and converted to a carbonyl complex which can be used to form the catalyst under the conditions of this process. However, when using such insoluble ruthenium compounds, they must first be solubilized before the effective operation of the process of this invention.

Ruthenium carbonyl compounds (which include ruthenium carbonyl hydrides or ruthenium carbonyl clusters) are already provided with a carbonyl ligand, and under the conditions of the reaction can be sufficiently changed to achieve the desired catalytic effect. Ruthenium salts such as those of organic acids can be employed in the practice of this invention to produce the catalyst. In addition to those ruthenium compounds described in the aforementioned Gresham patent, one may employ ruthenium compounds of bidentate ligands, allyl complexes, arene complexes, halides, and alkyl complexes. The choice of ruthenium compounds is varied and not critical to this invention. A number of ruthenium complexes are known to be more stable to the presence of carbon monoxide than other ruthenium compounds and the skilled worker can determine which particular ruthenium compound might take longer to initiate a reaction as the ruthenium catalyst than other ruthenium compounds. On that basis, one can select for the purposes of convenience the particular ruthenium compound to be utilized in forming the catalyst. However, ruthenium which is associated with an organic molecule or complexed with carbon monoxide is most readily solubilized so as to provide the ruthenium catalyst of this process.

The other component of the catalyst is a cocatalyst formed from a rhodium compound. The rhodium compound which is provided to the reaction is not necessarily in a form which will effectively activate the reaction catalyst even if it contains a carbon monoxide ligand bonded to it. It is believed that any of a host of rhodium-containing substances can be introduced into the reaction zone and under the operative conditions of the process (which of course includes hydrogen and carbon monoxide), the active rhodium compound can be generated *in situ*. Illustrative of rhodium-containing substances which can be conveniently introduced or placed in the synthesis zone include, for example, rhodium oxide ($Rh_2O_3$), tetrarhodium dodecacarbonyl, dirhodium octacarbonyl, hexarhodium hexadecacarbonyl ($Rh_6(CO)_{16}$), rhodium(II) formate, rhodium(II) acetate, rhodium(II) propionate, rhodium(II) butyrate, rhodium(II) valerate, rhodium(III) naphthenate, rhodium dicarbonyl acetylacetonate, rhodium tris(acetylacetonate), rhodium trihydroxide, indenylrhodium dicarbonyl, rhodium dicarbonyl (1-phenyl butane-1,3-dione), tris(hexane - 2,4 - dionato)rhodium(III), tris(heptane - 2,4 - dionato)rhodium(III), tris(1-phenyl butane - 1,3 - dionato)rhodium(III), tris(3 - methylpentane 2,4 - dionato)rhodium(III), tris(1 - cyclohexylbutane - 1,3 - dionato)rhodium(III), finely divided rhodium metal, rhodium metal and rhodium-containing compounds deposited on porous supports or carriers such as those exemplified previously. Under the conditions of the reaction the rhodium-containing substance is converted into a carbonyl compound which effectively activates the process to increase the production or selectivity of the process to ethylene glycol. The composition and structure of the rhodium carbonyl compound is not specifically known but is believed to be a monoatomic rhodium carbonyl structure, as distinguished from the rhodium carbonyl cluster chemistry above discussed in the background of the invention. In some instances, some modification of a rhodium cluster structure may be needed, possibly the destruction of such a cluster structure to a mononuclear rhodium carbonyl structure to achieve such a monoatomic rhodium cocatalyst. Factors in achieving the monoatomic rhodium carbonyl cocatalyst are the reaction parameters, the choice of solvent and, optionally, the Lewis base promoter that is employed. Because varied reaction conditions and solvents with or without promoters result in different amounts of the desired products of the process, and different rates, efficiencies and/or yields, it is presumed that each provides a different and distinct catalytic environment. The mononuclear rhodium carbonyl complexes believed present herein have stability greater than that normally associated with rhodium cluster compounds.

As characterized above, this process is operated as a homogeneous liquid phase mixture. The process is typically carried out in a solvent for the ruthenium catalyst and rhodium co-catalyst and the promoter. Thus the solvent is a liquid in which the catalyst (presumed to be a ruthenium carbonyl compound and a rhodium carbonyl compound and, the promoter) is soluble under the prescribed conditions of the reaction. The solvent may be solid at room temperature but should at least in part be a liquid under the conditions of reaction. Illustrative of suitable solvents herein are, e.g., water, ketones, esters including lactones, amides including lactams, sulfones, sulfoxides, halogenated hydrocarbons, aromatic hydrocarbons, and organic phosphine oxides. Illustrative of specific solvents encompassed by the above classes of polar solvents are, for example, aromatic hydrocarbons, e.g., benzene, toluene, xylene, naphthalene, alkylnaphthalene, ketones such as acetone, methyl ethyl ketone, cyclohexanone, cyclopentanone; esters such as methyl acetate, ethyl acetate, propyl acetate, butyl acetate, methyl propionate, ethyl butyrate, methyl laurate; lactams such as N-alkyl caprolactam, such as N-methylcaprolactam, N-alkyl pyrrolidinones such as N-methylpyrrolidinone; cyclic ureas such as N,N-dimethylimidazolidone; polyols such as ethylene glycol, glycerine, erythritol, polyalkylene glycol containing 2000 to 10000 repeating units; lactones such as gamma-butyrolactone; halogenated hydrocarbons such as chlorobenzene, chloroform, methylene chloride, 2,2-dichloropropane; amides such as dimethylformamide, dimethylacetamide, hexamethylphosphoramide; sulfones such as sulfolane, dimethylsulfone, substituted sulfolanes sulfoxides such as dimethylsulfoxide, diphenyl sulfoxide; organic phosphine oxides such as trialkylphosphine oxides and triarylphosphine oxides.

Illustrative of other suitable solvents are the ethers, cryptands. Illustrative of specific solvents encompassed by the above classes of complexing solvents are, for example, ethers such as tetrahydrofuran, tetrahydropyran, diethyl ether, 1,2-dimethoxybenzene, 1,2-diethoxybenene, the mono and dialkyl ethers of alkylene and polyalkylene glycols, such as ethylene glycol, of 1,2-propylene glycol, of 1,2-butylene glycol, of diethylene glycol, of di - 1,2 - propylene glycol, of triethylene glycol, of

pentaethylene glycol (such as triglyme, tetraglyme and pentaglyme), of di - 1,2 - butylene glycol, of oxyethylene-oxypropylene glycols, preferably those in which the alkylene group contains 2 and/or 3 carbon atoms in the divalent moiety, such as ethylene and 1,2-propylene; the cryptands such as described in U.S. Patent No. 4,111,975, as promoters in that case and the crown ethers (or Crown Ethers, as one may prefer) such as described in U.S. Patent No. 4,162,261, as solvents.

The choice of solvent in any particular case can be a complex decision. Some solvents such as the carboxylic acids may play a dual role in the practice of the process of this invention. They can act as a Lewis base promoter as well as the solvent. Other solvents which can play this dual function include, e.g., the crown ethers and the cryptands. In many instances, solvents react with the products of the reaction and such reactive solvents are considered useful in the practice of this invention because the derivative products obtained are an excellent source for the desired products of the reaction.

Mixtures of solvents may also be employed in the practice of the invention.

Because $H_2$ is supplied to the reaction, a hydride of ruthenium and/or rhodium can exist in the reaction system. There is no appreciation of the particular role that a hydride, if it exists, is playing in the reaction. It is believed that either too much or too little hydrogen present in the reaction will not favor the production of ethylene glycol.

Though the process of this invention is capable of providing a combination of ethylene glycol and methanol, in many instances one or more of them is formed to a greater extent and more ethylene glycol is formed relative to methanol than in prior processes and such is an important advantage of the instant process. Because ethylene glycol is the most valued of the products, its enhanced production relative to previous processes obviously makes this process attractive. A process which produces the same amount of ethylene glycol and produces less methanol will have more commercial attractiveness, assuming all other factors are equal.

At this time, no particular basis has been found for predicting whether any particular set of process conditions and reactants encompassed by this invention will produce ethanol except those that have already been established by experimentation. It has been found that certain process conditions, in fact all process conditions studied, produce less methanol than a process employing only a Ru catalyst although not all conditions are as effective in decreasing the production of methanol while increasing the production of ethylene glycol. The ability to make ethylene glycol preferentially to methanol may reside in the particular ruthenium catalyst, rhodium cocatalyst, the promoter, the solvent, and/or the temperature and pressure of reaction, but in all probability ethylene glycol production is dependent on a combination of all of these.

The relative amounts of carbon monoxide and hydrogen which are initially present in the reaction mixture can be varied over a wide range. In general, the molar ratio of $CO:H_2$ is in the range of from 40:1 to 1:40, suitably from 20:1 to 1:20, and preferably from 10:1 to 1:10. It is to be understood, however, that molar ratios outside the broadest of these may be employed. Substances or reaction mixtures which give rise to the formation of carbon monoxide and hydrogen under the reaction conditions may be employed instead of mixtures comprising carbon monoxide and hydrogen which are used in preferred embodiments the practice of the invention. For instance, the product alcohols are contemplated as obtainable by using mixtures containing carbon dioxide and hydrogen. Mixtures of carbon dioxide, carbon monoxide and hydrogen can also be employed. If desired, the reaction mixture can comprise steam and carbon monoxide.

The quantity of the ruthenium catalyst employed is not narrowly critical and can vary over a wide range. In general, the process is desirably conducted in the presence of a catalytically effective quantity of the active ruthenium species which gives a suitable and reasonable reaction rate when employed in combination with the rhodium cocatalyst. Reaction can proceed when employing as little as about $1 \times 10^{-6}$ weight percent, and even lesser amounts, of ruthenium based on the total weight of reaction mixture (i.e., the liquid phase mixture). The upper concentration limit can be quite high, e.g., about 30 weight percent ruthenium, and higher, and the realistic upper limit in practicing the invention appears to be dictated and controlled more by economics in view of the cost of ruthenium. Since the rate of conversion of synthesis gas may be dependent upon the concentration of ruthenium and rhodium employed, higher concentrations achieving higher rates, then larger concentrations of ruthenium may prove to be a most desirable embodiment of this invention. Depending on various factors such as the promoter, the partial pressures of carbon monoxide and hydrogen, the total operative pressure of the system, the operative temperature, the choice of solvent, and other considerations, a catalyst concentration of from $1 \times 10^{-3}$ to 20 weight percent ruthenium (contained in the complex catalyst) based on the total weight of reaction mixture, is generally desirable in the practice of the invention.

The quantity of rhodium cocatalyst employed is not narrowly critical and can vary over a wide range. In general, the process is desirably conducted in the presence of a catalytically effective quantity of the active rhodium species which gives a suitable and reasonable reaction rate when employed in combination with the ruthenium catalyst. Reaction can proceed when employing as little as about 100 parts per million by weight of rhodium based on the total weight of reaction mixture (i.e., the liquid phase mixture). A minimum quantity of rhodium appears to be required although the actual minimum quantity is not known at present. It is believed that the upper concentration limit can be quite high, e.g., about 30 weight percent rhodium, and higher, and the realistic upper limit in practicing the invention appears to be dictated and controlled

more by economics, the quantity of ruthenium catalyst employed, and the relative productivity of the process to a given product. Since the rate of conversion of synthesis gas to products may be dependent upon the concentration of ruthenium and rhodium employed, higher concentrations achieving higher rates, then large concentrations may prove to be a most desirable embodiment of this invention. Obviously a practical limit on the amount of rhodium employed is dictated by the cost of rhodium and the solubility of rhodium in the solvent. Depending on various factors such as the promoter, the partial pressures of carbon monoxide and hydrogen, the total operative pressure of the system, the operative temperature, the choice of solvent, and other considerations, a concentration of from $1 \times 10^{-3}$ to 20 weight percent rhodium (present as a rhodium carbonyl) based on the total weight of reaction mixture, is generally desirable in the practice of the invention. An important feature of the instant invention is the ability to employ relatively small amounts of the rhodium cocatalyst as compared to the ruthenium compound while achieving a rate to ethylene glycol greater than that rate achievable by use of either the ruthenium catalyst or rhodium cocatalyst alone. To the extent that such a combination of ruthenium and rhodium exhibit a greater rate to ethylene glycol the combination is synergistic in nature. The exact reason why the combined system is synergistic and achieves a rate to ethylene glycol greater than the sum of the individual rates is not clearly understood although the synergistic effect may involve the ability of the rhodium cocatalyst to be involved in a reaction with an intermediate formed by the ruthenium catalyst.

In practicing the invention the ratio of ruthenium catalyst rhodium cocatalyst is not narrowly critical and is generally selected to provide that amount of each catalyst which provides the synergistic combination. The ratio of ruthenium to rhodium is generally between 100 to 1 and 1 to 100 and is preferably selected to provide at least a slight molar excess of ruthenium compound over rhodium compound, i.e., the ratio of the number of ruthenium atoms to rhodium atoms is greater than 1. The molar ratio of rhodium to ruthenium is preferably between 1:1 and 1:100 and more preferably between 1:5 and 1:50. Although a molar ratio of rhodium to ruthenium less than 1 is preferably employed, such may or may not be ultimately preferred.

The temperature which may be employed in practicing the process may vary over a wide range of elevated temperatures. In general, the process can be conducted at a temperature between 50°C and 400°C and higher. Temperatures outside this stated range, though not excluded from the scope of the invention, do not fall within certain desirable embodiments of the invention. At the lower end of the temperature range, and lower, the rate of reaction to desired product becomes markedly slow. At the upper temperature range, and beyond, catalyst, solvent, or Lewis base promoter instability may occur. Notwithstanding these factors, reaction will continue and the products and/or their derivatives will be produced. Additionally, one should take notice of the equilibrium reaction for forming ethylene glycol:

$$2CO + 3H_2 \leftrightharpoons HOCH_2CH_2OH$$

At relatively high temperatures the equilibrium increasingly favors the left hand side of the equation. To drive the reaction to the formation of increased quantities of ethylene glycol, higher partial pressures of carbon monoxide and hydrogen are required. Processes based on correspondingly higher operative pressures, however, do not represent preferred embodiments of the invention in view of the high investment costs associated with erecting chemical plants which utilize high pressure utilities and the necessity of fabricating equipment capable of withstanding such enormous pressures. Preferred temperatures are between 100°C. and 350°C., and most desirably between 150°C. and 300°C.

The process is suitably effected over a wide superatmospheric pressure range. At pressures in the direction of and below about 34,5 bar abs. the rate of desired product formation is quite slow, and consequently, relatively faster reaction rates and/or higher conversions to the desired products can be obtained by employing higher pressures, e.g., pressures of at least about 1,9 bar abs. Pressures as high as 1380 to 3450 bar abs., and higher, can be employed but there is no apparent advantage in using such pressures, and any advantage that could be reasonably contemplated would be easily offset by the very unattractive plant investment outlay required for such high pressure equipment and the costs associated with such high pressure operations. Therefore, the upper pressure limitation is approximately 1035 bar abs. Effecting the process below about 1035 bar abs., especially below about 690 bar abs., results in significant cost advantages which are associated with lower pressure equipment requirements and operating costs. A suitable pressure range is from 34,5 bar abs. to 862,5 bar abs. The pressures referred to above represent the total pressure of hydrogen and carbon monoxide.

The pressure is effected for a period of time sufficient to produce the desired alkanols and/or derivatives thereof. In general, the residence time to produce the desired products can vary from minutes to a number of hours, e.g., from a few minutes to 24 hours, and longer. It is readily appreciated that the residence period (time) will be influenced to a significant extent by the reaction temperature, the concentration and choice of Lewis base promoter (if employed), rhodium source, ruthenium source, the total gas pressure and the partial pressure exerted by its components, the concentration and choice of solvent, and other factors. The synthesis of the desired product(s) by the reaction of hydrogen with carbon monoxide is suitably conducted under operative conditions which give reasonable reaction rates and/or conversions.

The process can be executed in a batch, semi-continuous, or continuous fashion. The reaction can be

conducted in a single reaction zone or a plurality of reaction zones, in series or in parallel, or it may be conducted intermittently or continuously in an elongated tubular zone or series of such zones. The material of construction should be such that it is inert during the reaction and the fabrication of the equipment should be able to withstand the reaction temperature and pressure. The reaction zone can be fitted with internal and/or external heat exchanger(s) to thus control undue temperature fluctuations, or to prevent any possible "runaway" reaction temperatures due to the exothermic nature of the reaction. In preferred embodiments of the invention, agitation means to vary the degree of mixing of the reaction mixture can be suitably employed. Mixing induced by vibration, shaker, stirrer, rotatory, oscillation, or ultrasonic, are all illustrative of the types of agitation means which are contemplated. Such means are available and well-known to the art. The catalyst precursor may be initially introduced into the reaction zone batchwise, or it may be continuously or intermittently introduced into such zone during the course of the synthesis reaction. Means to introduce and/or adjust the reactants, either intermittently or continuously, into the reaction zone during the course of the reaction can be conveniently utilized in the process especially to maintain the desired molar ratios of and the partial pressures exerted by the reactants.

As intimated previously, the operative conditions can be adjusted to optimize the conversion to the desired product, particularly ethylene glycol, and/or the economics of the process. In a continuous process, for instance, when it is preferred to operate at relatively low conversions, it is generally desirable to recirculate unreacted synthesis gas with/without make-up carbon monoxide and hydrogen to the reactor. Recovery of the desired product can be achieved by methods well-known in the art such as by distillation, fractionation, extraction, and the like. A fraction comprising the ruthenium and rhodium compounds generally contained in byproducts and/or the solvent, can be recycled to the reaction zone, if desired. All or a portion of such fraction can be removed for recovery of the ruthenium and rhodium values or regeneration thereof, if necessary. Fresh ruthenium and/or rhodium precursor, Lewis base promoter and/or solvent, can be intermittently added to the recycle stream or directly to the reaction zone, if needed.

Many embodiments of the ruthenium carbonyl catalyst, rhodium carbonyl cocatalyst, promoter and solvent combinations encompassed by this invention are sufficiently stable to allow repeated use of the ruthenium catalyst and rhodium cocatalyst. This is especially noted when the promoter is an alkali metal iodide or other iodide source. For example, the process of this invention can be continuously operated in a pressure reactor into which is continuously fed synthesis gas. The velocity of the synthesis gas may be employed at a sufficient rate to strip products of the reaction out of the reactor leaving behind in the reactor the ruthenium catalyst, rhodium catalyst, promoter and solvent combination. The products are separated from the unreacted synthesis gas and the synthesis gas is recycled to the reactor. The products, in this embodiment, are recovered free of ruthenium, rhodium, promoter and solvent. In this embodiment, the ruthenium and rhodium catalyst need not be removed from the reactor to a recovery zone for separating product. Thus a catalyst treatment step is avoided. The examples below depict batch reactions; however, the above continuous gas recycle process can be operated in a similar manner. That is, the batch reactor simulates the continuous reactor except for the gas sparging and continuous gas recycle.

Although this invention has been described with respect to a number of details, it is not intended that this invention should be limited thereby. Moreover, the examples which follow are intended solely to illustrate a variety of, including the most favorable, embodiments of this invention and are not intended in any way to limit the scope and the intent of this invention.

Experimental procedure

The following procedure was employed in the examples recorded below.

A 150 ml capacity stainless steel reactor capable of withstanding pressures up to 3,000 bar was charged with a mixture of solvent, catalyst precursor, and a promoter, as indicated below. The reactor was sealed and charged with carbon monoxide to a pressure of 34,5 bar. In some cases the gaseous contents of the reactor are vented to remove oxygen. In these cases the reactor is then repressurized to about 34,5 bar (This venting procedure may be repeated if desired.)

Heat was then applied to the reactor and its contents, (initially at 55°C or as otherwise indicated); when the temperature of the mixture inside the reactor reached the designated reaction temperature as measured by a suitably placed thermocouple, addition of carbon monoxide and hydrogen ($H_2$:CO equals the designated mole ratio) was made to bring the pressure to the specified reaction pressure. The temperature was maintained at the desired value for the reported time period. During this period of time, additional carbon monoxide and hydrogen were added whenever the pressure inside the reactor dropped by more than about 34,5 bar over the entire reaction period.

After the reaction period, the reaction vessel was cooled to room temperature, the reaction vessel vented and the reaction products removed. Analysis of the reaction mixture was made by gas chromatographic methods.

The various rates set forth in the following examples are average rate for the particular product and are determined by measuring the net production of product for the reaction period and assuming a nominal reaction volume of 75 ml.

Example 1—5

Examples 1 and 2 are comparative examples wherein either rhodium or ruthenium were not employed

and are included in Table I for comparison purposes only. Examples 3—5 are examples according to this invention.

Examples 1 and 2 were carried out according to the above-described experimental procedure by adding $Rh(CO)_2(acac)$ [acac=acetylacetonate] or $Ru_3(CO)_{12}$ to 75 milliliters of N-methylpyrrolidinone (NMP) and 18 millimoles of sodium iodide. Examples 3—5 were carried out by adding $Rh(CO)_2(acac)$ to a mixture containing $Ru_3(CO)_{12}$ (in an amount as indicated in Table I), 75 milliliters of a N-methylpyrrolidinone and 18 millimole of sodium iodide. Examples 1—5 were carried out under pressure of 862,5 bar of a 1:1 mixture of $H_2$ to CO at a temperature of 230°C. The results are set forth in Table I and are graphically displayed in Figure 1.

TABLE I

| Example | Ru[1] | Rh[2] | MeOH[3] | Glycol[4] |
|---|---|---|---|---|
| 1 (comp.) | 6 | 0 | 4.98 | 0.71 |
| 2 (comp.) | 0 | 3.0 | 0.06 | 0.18 |
| 3 | 6 | 0.5 | 4.00 | 1.89 |
| 4 | 6 | 1.0 | 3.20 | 2.04 |
| 5 | 6 | 3.0 | 3.45 | 2.89 |

[1] millimoles of ruthenium
[2] millimoles of rhodium
[3] rate of production of methanol in mole/l · h
[4] rate of production of ethylene glycol in mole/l · h

Examples 6—10

Examples 6 and 7 are comparative examples and examples 8—10 are examples carried out according to the invention. Examples 6—10 were carried out similarly to examples 1—5 except that 90 millimoles of NaI were employed, the pressure was 414 bar and the temperature was 230°C. The results are set forth in Table II and graphically displayed in Figure 2.

TABLE II

| Example | Ru[1] | Rh[2] | MeOH[3] | Glycol[4] |
|---|---|---|---|---|
| 6 (comp.) | 15 | 0 | 4.00 | 0.25 |
| 7 (comp.) | 0 | 6.0 | 0.065 | 0.08 |
| 8 | 15 | 2.0 | 3.19 | 0.57 |
| 9 | 15 | 3.0 | 2.91 | 0.64 |
| 10 | 15 | 6.0 | 3.13 | 0.94 |

[1] millimoles of ruthenium
[2] millimoles of rhodium
[3] rate of production of methanol in mole/l · h
[4] rate of production of ethylene glycol in mole/l · h

Examples 11—15

Examples 12—15 were carried out according to the process of this invention. Example 11 is a comparative example wherein only rhodium is employed. Examples 11—15 were carried out according to the above-described experimental procedure by adding $Ru_3(CO)_{12}$ to a mixture comprising 75 milliliters of N-methylpyrrolidinone, 90 millimoles of NaI and 2.0 millimoles of rhodium (added as $Rh(CO)_2$ (acac)). The process was carried out under an atmosphere of hydrogen and carbon monoxide (1:1 molar ratio) under a pressure of 862,5 bar and at a temperature of 230°C. The results of examples 11—15 are set forth in Table III.

TABLE III

| Example | Ru[1] | Rh[2] | MeOH[3] | Glycol[4] |
|---|---|---|---|---|
| 11 (comp.) | 0 | 2 | 0.07 | 0.05 |
| 12 | 3 | 2 | 4.34 | 1.48 |
| 13 | 6 | 2 | 7.79 | 2.77 |
| 14 | 9 | 2 | 15.4 | 5.62 |
| 15 | 12 | 2 | 25.2 | 6.73 |

[1] millimoles of ruthenium
[2] millimoles of rhodium
[3] rate of production of methanol in mole/l · h
[4] rate of production of ethylene glycol in mole/l · h

Examples 16—24

Examples 16—24 were carried out according to the above-described Experimental Procedure except that the promoter in each example, the amount of ruthenium compound and the amount of rhodium compound were as set forth in Table IV using 75 milliliters of N-methylpyrrolidinone as the solvent and a pressure of 862,5 bar of 1:1 mixture of $H_2$ and CO at a temperature of 230°C. Examples 17, 18, 20 and 21 are comparative examples.

TABLE IV

| Example | Ru[1] | Rh[2] | Promoter[3] | mmole[4] | Glycol[5] | MeOH[6] |
|---|---|---|---|---|---|---|
| 16 | 6 | 2 | NaOAc | 18 | 0.05 | 0.75 |
| 17 | 6 | — | NaOAc | 18 | 0.05 | 1.11 |
| 18 | — | 2 | NaOAc | 18 | 0.02 | 0.045 |
| 19 | 6 | 2 | $K_2HPO_4$ | 18 | 0.065 | 0.65 |
| 20 | 6 | — | $K_2HPO_4$ | 18 | 0.02 | 1.06 |
| 21 | — | 2 | $K_2HPO_4$ | 18 | 0.02 | 0.05 |
| 22 | 6 | 2 | NaI | 18 | 1.09 | 1.46 |
| 23 | 6 | 2 | NaBr | 18 | 0.53 | 0.65 |
| 24 | 6 | 2 | NaCl | 18 | 0.30 | 0.89 |

[1] Millimoles of ruthenium.
[2] millimoles of rhodium.
[3] NaOAc=sodium acetate;
[4] millimoles of promoter.
[5] rate of production of ethylene glycol in mole/l · h
[6] rate of production of methanol in mole/l · h

**Claims**

1. The process for making alkanols including methanol and ethylene glycol directly by reacting in the homogeneous liquid phase a mixture of hydrogen and carbon monoxide in the presence of a ruthenium carbonyl catalyst, a rhodium carbonyl cocatalyst and a solvent at 50—400°C and a pressure of 34.5 to 862.5 bar abs. characterized in that a combination of a non-ionic, polar solvent and an alkalimetal halide promoter is used.

2. The process of claim 1 wherein the solvent is an ether or N-methylpyrrolidinone.

3. The process of claim 1 or 2 wherein the amount of promoter ranges from 0.1 mole to $10^6$ moles for each gram atom of ruthenium present.

4. The process of claim 1 to 3 wherein the molar ratio of ruthenium to rhodium is between 100 to 1 and 1 to 100.

5. The process of claim 4 wherein the molar ratio of rhodium to ruthenium is between 1 to 5 and 1 to 50.

**Patentansprüche**

1. Verfahren zur direkten Herstellung von Alkoholen, einschließlich Methanol und Ethylenglykol, durch Umsetzung von Wasserstoff und Kohlenmonoxid in homogener, flüssiger Phase in Gegenwart eines Rutheniumcarbonylkatalysators, eines Rhodiumcarbonylcokatalysators und eines Lösungsmittels bei 50 bis 400°C unter einem Druck von 34,5 bis 862,5 bar abs, dadurch gekennzeichnet, daß man ein nicht ionisches polares Lösungsmittel und einen Promotor in Form eines Alkalihalogenids verwendet.

2. Verfahren nach Anspruch 1, worin das Lösungsmittel ein Ether oder N-Methylpyrrolidinon ist.

3. Verfahren nach Anspruch 1 oder 2, worin die Promotormenge je g-Atom Ruthenium 0,1 bis $10^6$ mol beträgt.

4. Verfahren nach Anspruch 1 bis 3, worin das Molverhältnis Ruthenium zu Rhodium 100:1 bis 1:100 beträgt.

5. Verfahren nach Anspruch 4, worin das Molverhältnis Rhodium zu Ruthenium 1:5 bis 1:50 beträgt.

**Revendications**

1. Procédé de production d'alcanols comprenant le méthanol et l'éthylèneglycol, par réaction directe en phase liquide homogène d'un mélange d'hydrogène et d'oxyde de carbone en présence d'un catalyseur au ruthéniumcarbonyle, d'un cocatalyseur au rhodium-carbonyle et d'un solvant à 50—400°C et sous une pression absolue de 34,5 à 862,5 bars, caractérisé en ce qu'on utilise conjointement un solvant polaire non ionique et un activateur qui est un halogénure de métal alcalin.

2. Procédé suivant la revendication 1, dans lequel le solvant est un éther ou la N-méthylpyrrolidinone.

3. Procédé suivant la revendication 1 ou 2, dans lequel la quantité d'activateur va de 0,1 à $10^6$ moles pour chaque atome-gramme de ruthénium présent.

4. Procédé suivant les revendications 1 à 3, dans lequel le rapport molaire du ruthénium au rhodium va de 100:1 à 1:100.

5. Procédé suivant la revendication 4, dans lequel le rapport molaire du rhodium au ruthénium va de 1:5 à 1:50.

FIG. 1

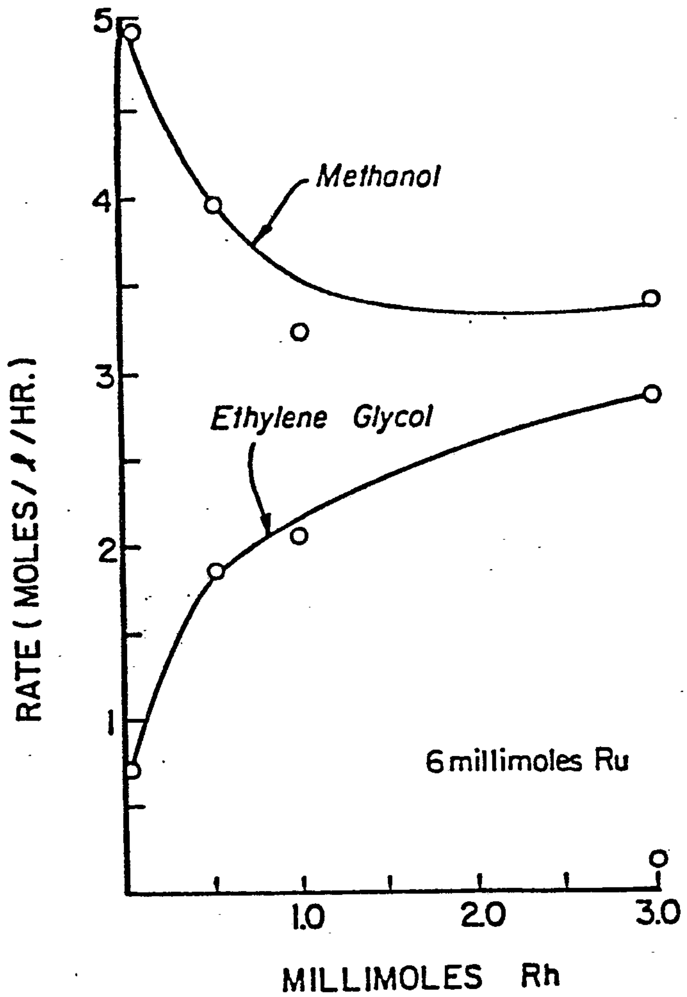
5
4
3
2
1
RATE ( MOLES / ℓ / HR. )
Methanol
Ethylene Glycol
6 millimoles Ru
1.0
2.0
3.0
MILLIMOLES Rh

FIG. 2

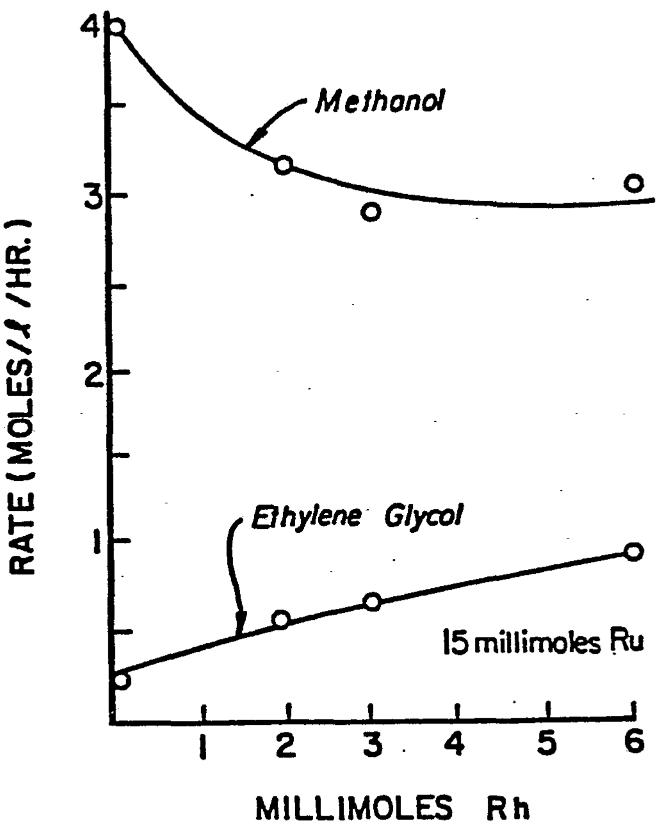
4
3
2
1
RATE (MOLES/ℓ/HR.)
Methanol
Ethylene Glycol
15 millimoles Ru
1
2
3
4
5
6
MILLIMOLES Rh